# EUROPEAN PATENT APPLICATION

(11) **EP 4 324 826 A1**
(43) Date of publication of application: **21.02.2024**
(21) Application number: 22788452.5
(22) Date of filing: 13.04.2022
(51) Int. Cl.: C07D 401/12, A61K 31/454, A61P 25/00, A61P 37/00

(54) **CRYSTALLINE FORM OF SPHINGOSINE-1-PHOSPHATE RECEPTOR AGONIST**

(30) Priority: 14.04.2021 KR 20210048825
(71) Applicant: LG Chem, Ltd., Seoul 07336 (KR)
(72) Inventor: KIM, Ji Yoon, Seoul 07796 (KR); CHUN, Seul Ah, Seoul 07796 (KR); KIM, Sung Wook, Seoul 07796 (KR); KIM, Sung Won, Seoul 07796 (KR); KIM, Ki Dae, Seoul 07796 (KR); LEE, Soo Min, Seoul 07796 (KR)
(74) Representative: Hoffmann Eitle
(86) International application number: PCT/KR2022/005374
(87) International publication number: WO 2022/220597

(57) **Abstract**

The present invention relates to a crystalline form of a sphingosine-1-phosphate receptor agonist and, more particularly, to a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid of chemical formula 1 or a pharmaceutically acceptable salt thereof.

## Description

### TECHNICAL FIELD

The present invention relates to a crystalline form of sphingosine-1-phosphate receptor agonist. More specifically, the present invention relates to a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 1 or a pharmaceutically acceptable salt thereof:

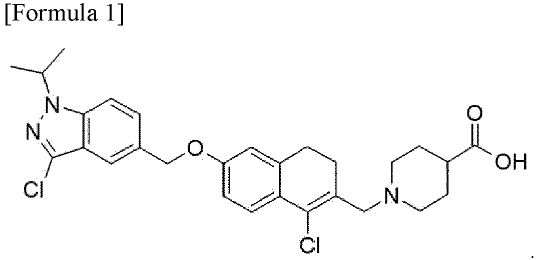

### BACKGROUND ART

Sphingosine-1-phosphate (S1P) is produced via an intracellular ceramide pathway, in which ceramide is the starting material. Ceramide is produced via two pathways, the first of which is a *de novo* biosynthetic pathway. Ceramide is also produced by the degradation of sphingomyelin, a cell membrane constituent, in a cell. The S1P level in each tissue is controlled by two biosynthetic sphingosine kinases (SphKs) and two biodegradable S1P phosphatases (S1P lyase and lysophospholipid phosphatases). S1P-which is produced via phosphorylation of sphingosine by sphingosine kinase-is known to mediate various cellular responses, such as cell proliferation, cytoskeletal organization and migration, adherence- and tight junction assembly, and morphogenesis. S1P exists as a combined form with plasma protein including albumin at high level (100 - 1,000 nM) in plasma, while it is at a low level in tissues.

S1P binds with S1P receptor, a G-protein coupled receptor, to show various biological functions. As S1P receptor sub-types, S1P1 to S1P5 are known up to now and are named endothelial differentiation gene (EDG) receptors 1, 5, 3, 6 and 8, respectively. The S1P receptors are known to be involved in various biological functions such as leukocyte recirculation, neural cell proliferation, morphological changes, migration, endothelial function, vasoregulation and cardiovascular development.

Meanwhile, the investigation of physical and chemical properties of a new drug is necessary for efficient and successful development of the new medicine. Specifically, by studying the presence of polymorphs and pseudopolymorphs of the drug and differences in physical and chemical properties between respective polymorphs the preferable crystal form of the drug can be selected in view of the pharmaceutical aspect (Remington's Pharmaceutics, Chapter 75 Preformulation); (Byrn, S.R., Solid State Chemistry of Drugs, Academic Press, New York, 1982). When the polymorphs are present in the solution, they are chemically identical, but in the solid state they respectively have definitely different X-ray diffraction patterns and exhibit differences in various physical and chemical properties. Specifically, respective polymorphs can have differences in bioavailability due to the differences in dissolution rates, and exhibit unexpected properties in the aspect of thermodynamic stability.

When a certain drug is present in the form of polymorphs, the crystalline forms having different structures can be obtained depending on the conditions of recrystallization such as recrystallizing solvent, drug concentration, heating and cooling rates, temperature, stirring rate, and the like, during the procedures for preparing the drug. Therefore, in order to obtain the same crystalline form a special attention is required for the management of manufacturing procedures. The difference in the crystal structure can be distinguished by X-ray diffraction pattern. Since in hydrates only physical properties such as crystallinity, hygroscopic property, melting point, solubility, dissolution rate, etc. are changed without any change of chemical properties providing pharmacological effects, they have a very important significance in the pharmaceutical aspect, like polymorphs (Morris, K. R. et al., Int. J. Pharm., 108, 1994, 15-206).

The knowledge which is understood up to date from various references relating to the technical field to which the present invention belongs is that there is no general tendency for the improvement of pharmaceutical properties including drug stability, hygroscopic property, etc. Ultimately, determination of the forms having the optimal pharmaceutical properties for respective compounds must be made through continuous study case by case, and it cannot be expected and belongs to the experimental area which can be confirmed only through repeated experiments.

### DISCLOSURE OF INVENTION

### TECHNICAL PROBLEM

The present invention is intended to provide a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 1 or a pharmaceutically acceptable salt thereof having excellent pharmaceutical properties:

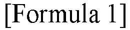

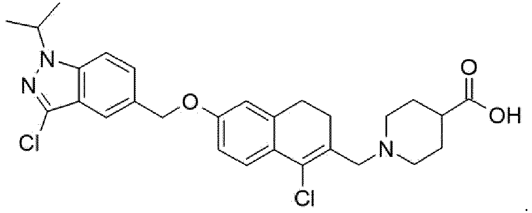

### SOLUTION TO PROBLEM

In order to solve the above technical problem, the present invention provides a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof.

In addition, the present invention provides a pharmaceutical composition comprising the crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as an active ingredient, together with a pharmaceutically acceptable carrier.

The present invention is described in detail hereinafter.

According to one aspect of the present invention, there is provided a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof having 3 or more, 5 or more, 7 or more, 9 or more, or 10 or more characteristic peaks (2θ) selected from the following X-ray diffraction pattern spectrum: 9.15±0.2°, 12.19±0.2°, 13.68±0.2°, 14.48±0.2°, 15.37±0.2°, 15.93±0.2°, 17.32±0.2°, 19.53±0.2°, 21.56±0.2°, 22.64±0.2°, 23.20±0.2°, 23.66±0.2°, 24.51±0.2°, 27.12±0.2°, 27.60±0.2° and 30.87±0.2°. Hereinafter, the above crystalline form refers to as "Crystalline Form A."

In one embodiment according to the present invention, the Crystalline Form A has 3 or more, 5 or more, 7 or more, 9 or more, or 10 or more characteristic peaks (2θ) selected from the following X-ray diffraction pattern spectrum: 9.15±0.1°, 12.19±0.1°, 13.68±0.1°, 14.48±0.1°, 15.37±0.1°, 15.93±0.1°, 17.32±0.1°, 19.53±0.1°, 21.56±0.1°, 22.64±0.1°, 23.20±0.1°, 23.66±0.1°, 24.51±0.1°, 27.12±0.1°, 27.60±0.1° and 30.87±0.1°.

When the Crystalline Form A is subjected to thermogravimetric analysis, a weight loss of about 0.7% is observed from ambient temperature to 100°C, and a dramatic change in the slope of the TGA thermogram is observed at about 230°C, which are most likely related to degradation of the material (Figure 2). When the Crystalline Form A is analyzed using differential scanning calorimetry (DSC), an endotherm is observed at about 221°C (onset) (Figure 3). Based on hot stage microscopy (HSM) observation (melting range: 217.1-226.4°C), the endotherm is attributed to the melting point of the material (Figure 5). According to the HSM observation, melting occurred simultaneously with decomposition. As a result of dynamic vapor sorption (DVS) analysis of the Crystalline Form A, a weight increase of 2.58% during sorption from 5% to 95%RH and a weight loss of 2.49% during desorption from 95% to 5%RH are observed (Figure 6). The solid retrieved after DVS shows XRPD patterns similar to the initial ones. Under accelerated and severe conditions, the Crystalline Form A is physically/chemically stable for 2 weeks.

According to another aspect of the present invention, there is provided a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof having 3 or more, 5 or more, 7 or more, 9 or more, or 10 or more characteristic peaks (2θ) selected from the following X-ray diffraction pattern spectrum: 8.87±0.2°, 12.53±0.2°, 13.40±0.2°, 14.10±0.2°, 15.22±0.2°, 15.64±0.2°, 16.09±0.2°, 17.06±0.2°, 17.62±0.2°, 18.91±0.2°, 20.67±0.2°, 21.05±0.2°, 22.64±0.2°, 23.59±0.2°, 24.22±0.2°, 25.52±0.2°, 26.05±0.2° and 27.09±0.2°. Hereinafter, the above crystalline form refers to as "Crystalline Form D."

In one embodiment according to the present invention, the Crystalline Form D has 3 or more, 5 or more, 7 or more, 9 or more, or 10 or more characteristic peaks (2θ) selected from the following X-ray diffraction pattern spectrum: 8.87±0.1°, 12.53±0.1°, 13.40±0.1°, 14.10±0.1°, 15.22±0.1°, 15.64±0.1°, 16.09±0.1°, 17.06±0.1°, 17.62±0.1°, 18.91±0.1°, 20.67±0.1°, 21.05±0.1°, 22.64±0.1°, 23.59±0.1°, 24.22±0.1°, 25.52±0.1°, 26.05±0. 1 ° and 27.09±0.1°.

In one embodiment according to the present invention, the pharmaceutically acceptable salt may be selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid. In one embodiment according to the present invention, the pharmaceutically acceptable salt may be hydrochloric acid.

According to still another aspect of the present invention, there is provided a pharmaceutical composition comprising the Crystalline Form A and/or Crystalline Form D of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof, together with a pharmaceutically acceptable carrier.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethylsulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the Crystalline Form A and/or Crystalline Form D of the compound of Formula 1 or a pharmaceutically acceptable salt thereof-is mixed with selected pharmaceutically acceptable carriers considering the dosage form to be prepared. For example, the pharmaceutical composition of the present invention can be formulated as injections, oral preparations and the like, as needed.

The crystalline form of the compound of Formula 1 of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The crystalline form of the compound of Formula 1 of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the crystalline form of the compound of Formula 1 of the present invention with at least one carrier selected from inert diluents such as sucrose, lactose or starch, lubricants such as magnesium stearate, disintegrating agents, binders and the like. In addition, it may be formulated as a transdermal dosage form-for example, as a lotion, ointment, gel, cream, patch or spray.

The pharmaceutical composition according to the present invention is suitable for preventing or treating diseases related to sphingosine-1-phosphate receptor. In one embodiment according to the present invention, the pharmaceutical composition may be used in the treatment of autoimmune disease including multiple sclerosis. In one embodiment according to the present invention, the pharmaceutical composition may be used in the prevention or treatment of a disease caused by undesired lymphocyte infiltration related to sphingosine-1-phosphate. In one embodiment according to the present invention, the pharmaceutical composition may be used in the prevention or treatment of immunoregulation disorder. In one embodiment according to the present invention, examples of the immunoregulation disorder may be autoimmune disease or chronic inflammatory disease selected from the group consisting of systemic lupus erythematosus, chronic rheumatoid arthritis, inflammatory bowel diseases, multiple sclerosis, amyotrophic lateral sclerosis (ALS), arteriosclerosis, atherosclerosis, scleroderma and autoimmune hepatitis, but are not limited thereto.

Herein, the term "prevention" refers to reducing or eliminating the possibility of contracting a disease.

Herein, the term "treatment" is used to mean deterring, delaying or ameliorating the progress of diseases in a subject exhibiting symptoms of diseases.

### EFFECTS OF THE INVENTION

The Crystalline Form A and/or Crystalline Form D of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof have pharmacological activity as a sphingosine-1-phosphate receptor agonist and at the same time have excellent pharmaceutical properties such as stability-for example, thermal stability and storage stability.

### BRIEF DESCRIPTION OF DRAWINGS

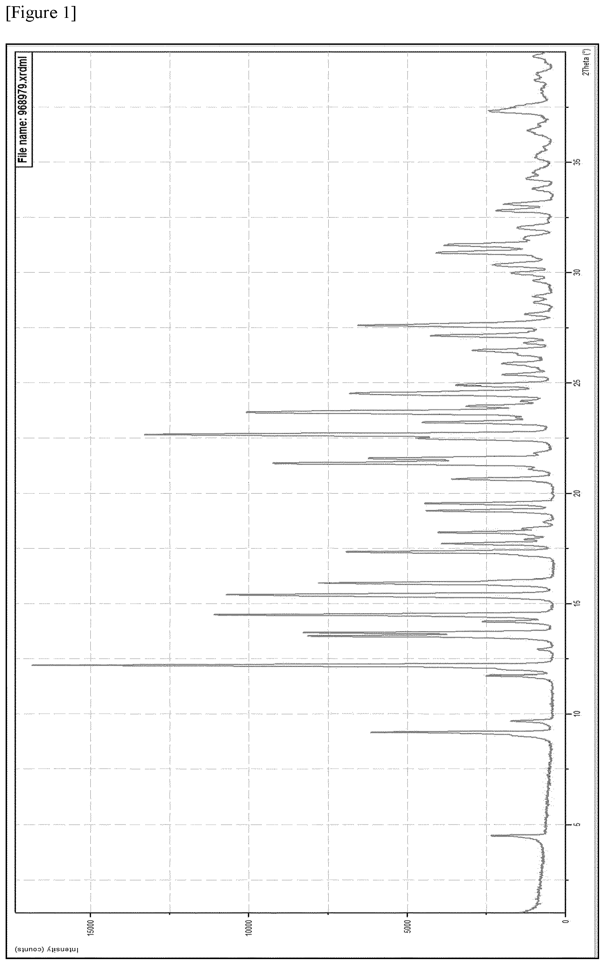

Figure 1 is an X-ray powder diffraction (XRPD) spectrum of Crystalline Form A.

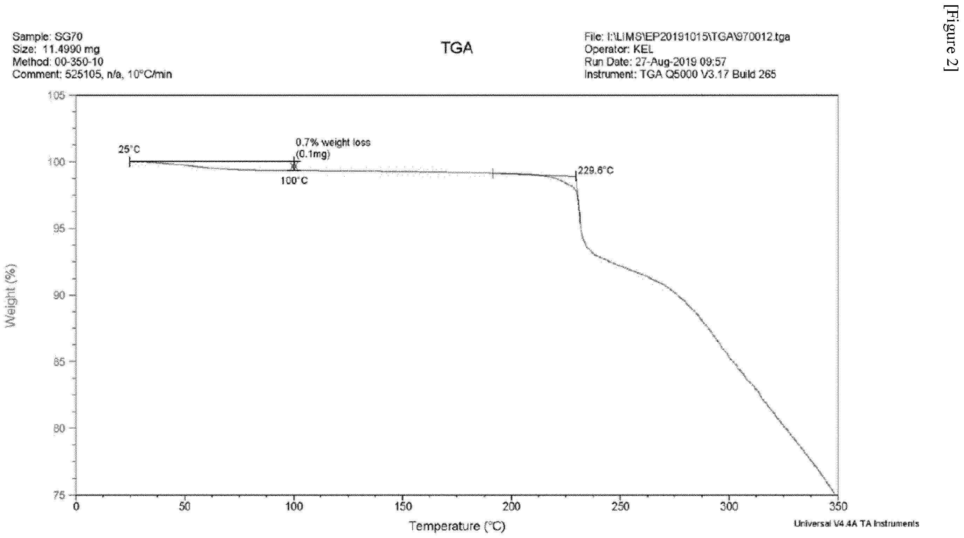

Figure 2 is a thermogravimetric analysis (TGA) result of Crystalline Form A.

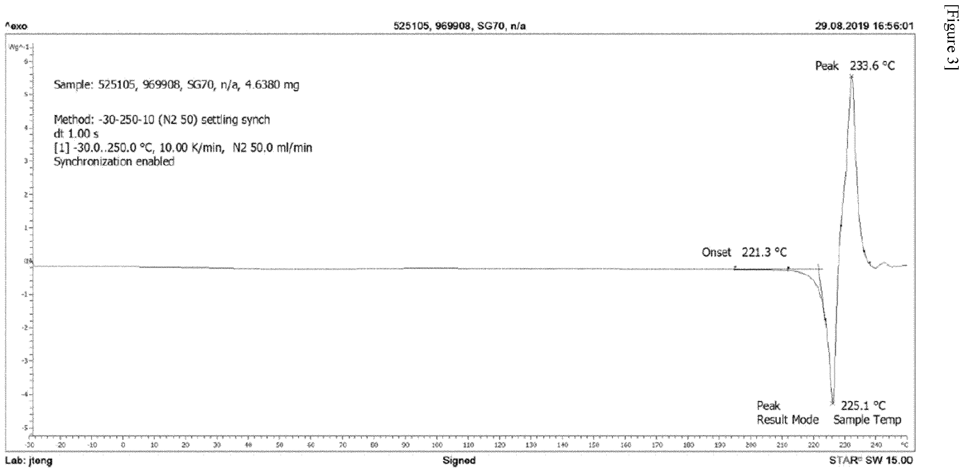

Figure 3 is a differential scanning calorimetry (DSC) analysis result of Crystalline Form A.

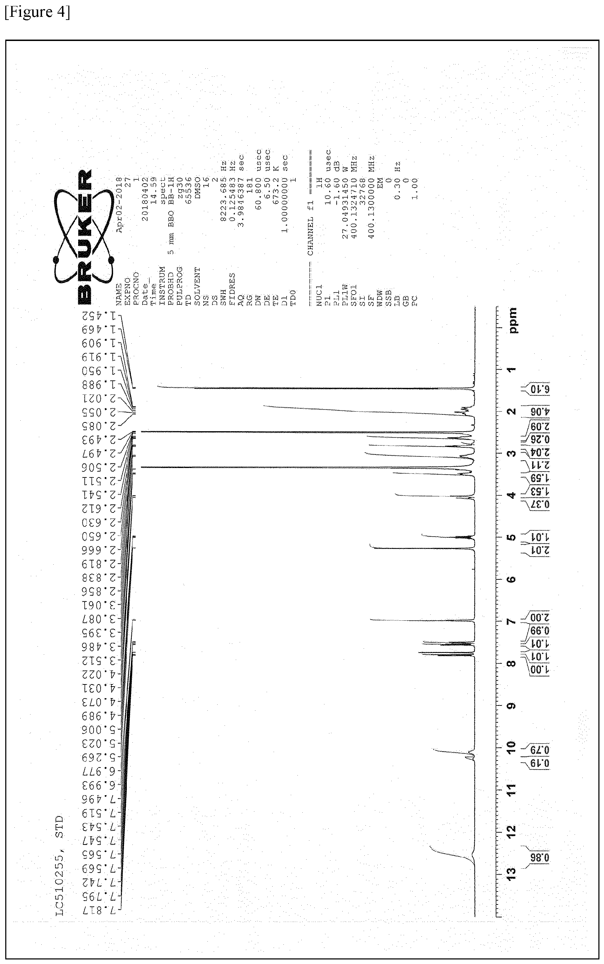

Figure 4 is an NMR spectrum of Crystalline Form A.

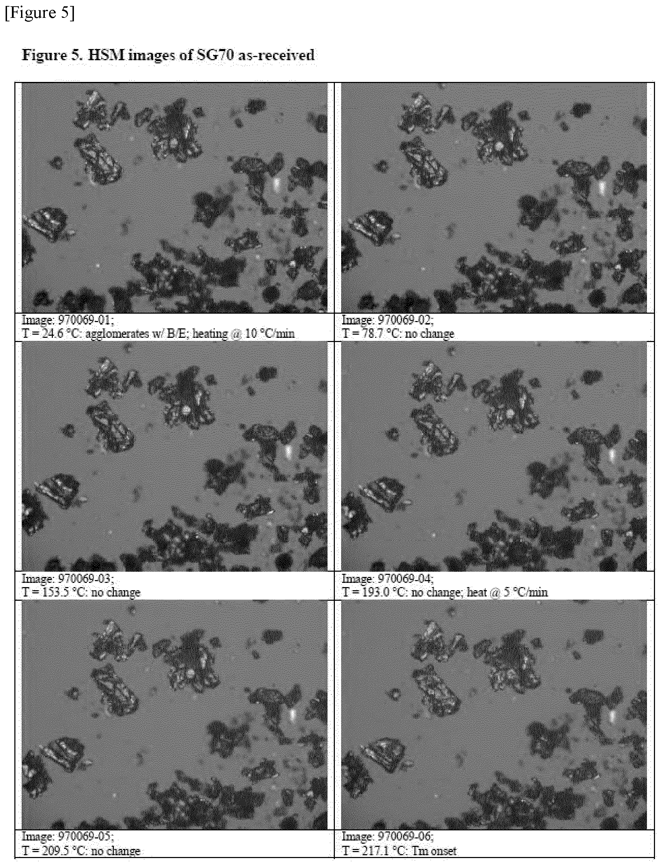

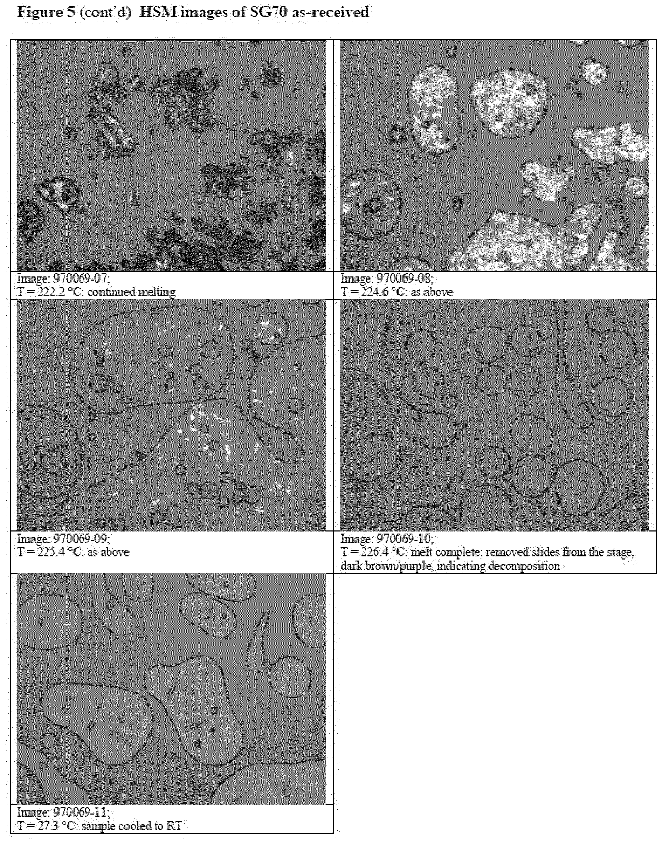

Figure 5 is a hot stage microscopy result of Crystalline Form A.

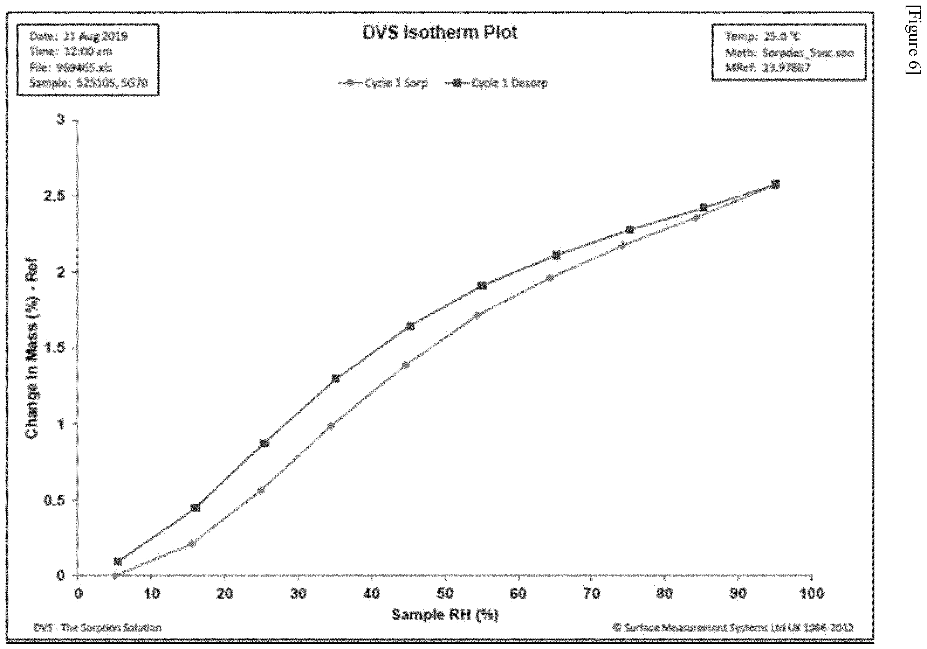

Figure 6 is a dynamic vapor sorption analysis result of Crystalline Form A.

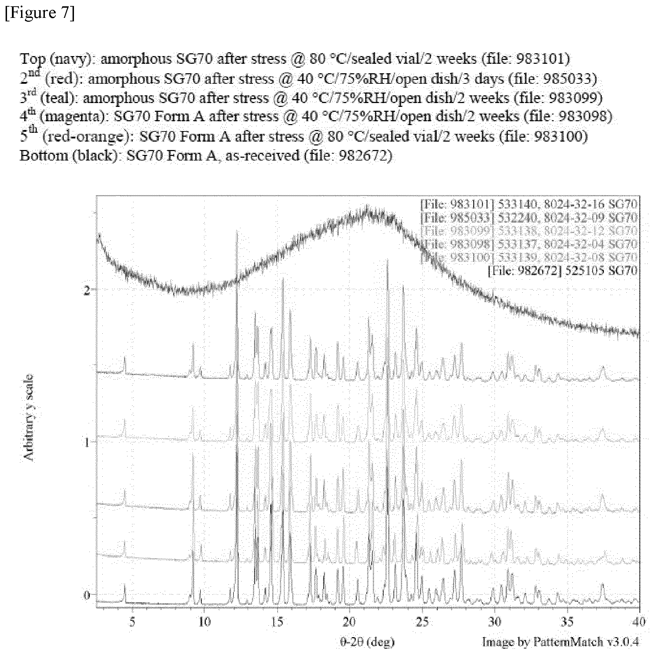

Figure 7 is a physical stability (XRPD) analysis result of Crystalline Form A.

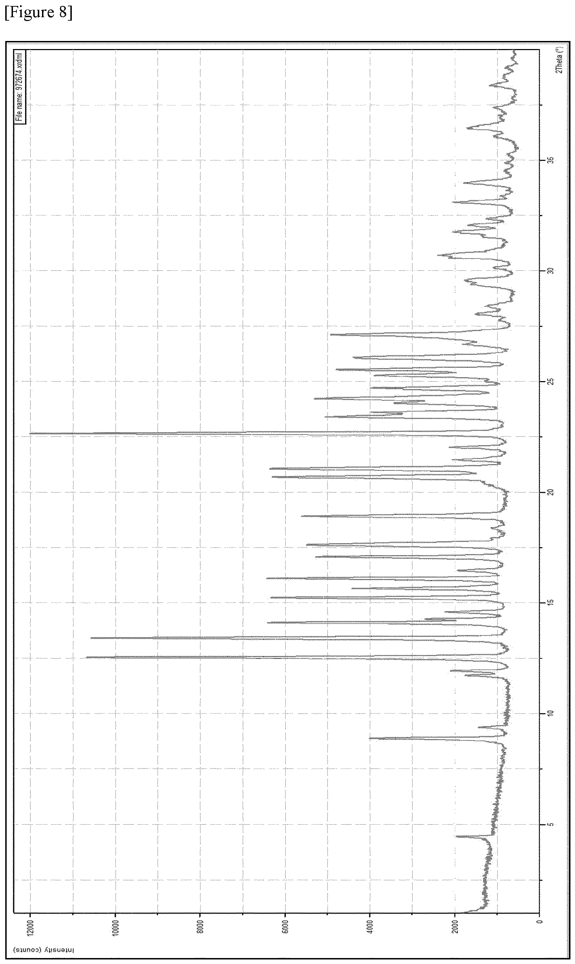

Figure 8 is an X-ray powder diffraction (XRPD) spectrum of Crystalline Form D.

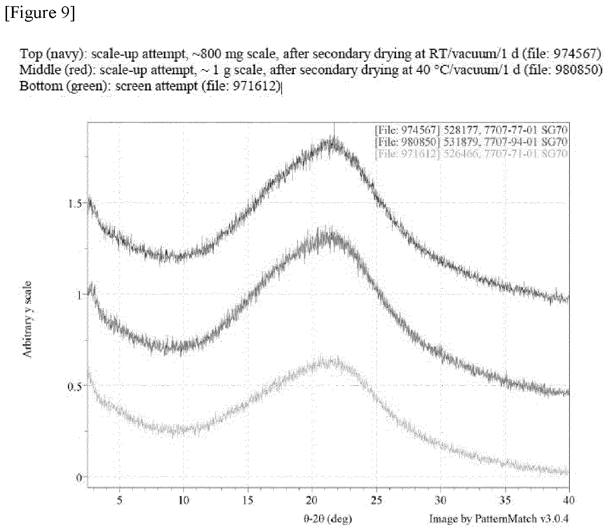

Figure 9 is an X-ray powder diffraction (XRPD) spectrum of amorphous form.

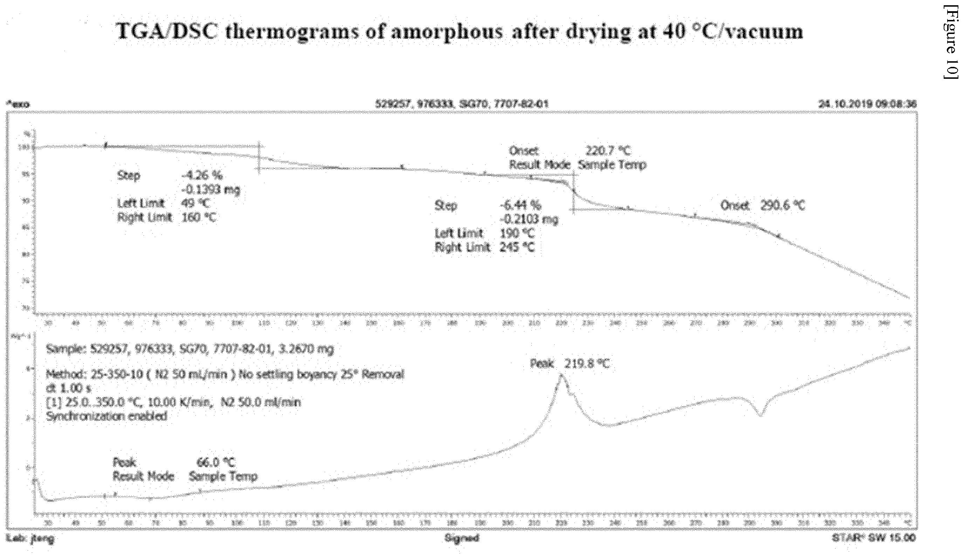

Figure 10 is thermogravimetric analysis (TGA)/differential scanning calorimetry (DSC) analysis results of amorphous form.

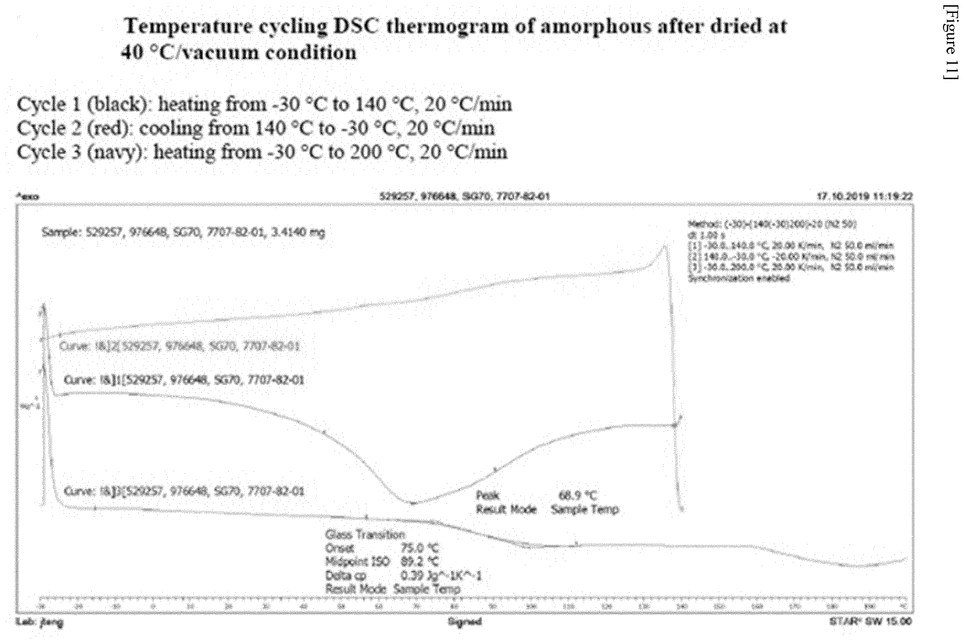

Figure 11 is a temperature cycling DSC result of amorphous form.

### MODE FOR THE INVENTION

Hereinafter, the present invention is explained in more detail with the following examples. However, it must be understood that the protection scope of the present invention is not limited to the examples.

### Preparation Example: Synthesis of 1-[1-chloro-6-(3-chloro-1-isopropyl-1H-indazol-5-ylmethoxy)-3,4-dihydro-naphthalen-2-ylmethyl]-piperidine-4-carboxylic acid hydrochloride

1-[1-Chloro-6-(3-chloro-1-isopropyl-1H-indazol-5-ylmethoxy)-3,4-dihydro-naphthalen-2-ylmethyl]-piperidine-4-carboxylic acid ethyl ester was synthesized according to the method described in Preparation Example 153-1 of International Publication No. WO 2014/129796 A1, the ester was hydrolyzed with NaOH, acidified with HCl, and then crystallization was carried out to obtain the hydrochloride form (hereinafter referred to as "Compound 1").

### Example 1: Preparation of Crystalline Form A

Compound 1 (226 g, 0.46 mol), ethanol (1.13 L, 5-fold), water (0.57 L, 2.5-fold) and NaOH (32 g, 0.80 mol) were added to a reactor. After heating at an internal temperature of 45°C for 60 minutes, the internal temperature was cooled to 28°C. After dichloromethane (DCM) (340 mL 1.5-fold) was added to the reaction mixture, 6 N HCl (167 ml, 1.00 mol) was slowly added dropwise over 50 minutes to acidify the pH of the solution to 2.5, and then ethyl acetate (EtOAc) (0.23 L, 1-fold) was added to proceed with crystallization. After cooling the internal temperature to 5°C, the obtained product was kept for 30 minutes, filtered and washed twice with water (1.13 L, 5-fold) and once with methyl tert-butyl ether (MTBE) (0.50 L, 3-fold) to obtain Crystalline Form A (156 g, two step yield: 69.0%).

### Example 2: Analysis of Crystalline Form A

### (1) XRPD (x-ray powder diffraction)

XRPD analysis was carried out by using a PANalytical X'pert Pro diffractometer with an incident beam of Cu radiation. After compacting about 20-30 mg of the sample to have a flat surface on the glass sample holder, the generator of the equipment was set to 45 kV (acceleration voltage) and 40 mA (filament emission), and then the measurement was conducted with reflection mode (not-spin). Bragg angles (2θ) in the range of 4 to 40° were measured with 0.026° of step size and 51 seconds of time per step conditions. XRPD patterns were classified and processed using HighScore Plus 2.2c software, and the results are represented in Figure 1 and Table 2.

### (2) TGA (thermogravimetric analysis)

TGA was carried out by using a TA Instruments Q5000 IR thermogravimetric analyzer. The sample was sealed and a pin-hole was punched into the sealed cap, and then the prepared sample was inserted into a TG furnace, which was heated under nitrogen from 25°C to a maximum of 350°C at a rate of 10°C/min. The results are represented in Figure 2.

### (3) DSC (differential scanning calorimetry)

DSC was carried out by using a Mettler-Toledo DSC 3+ differential scanning calorimeter. The sample was placed in a sealed aluminum DSC pan, and the weight was accurately recorded. Then, the pan lid was pierced, and the sample was placed into the DSC cell for measurement. Heating under nitrogen was performed from 30°C to a maximum of 250°C at a rate of 10°C/min. The results are represented in Figure 3.

### (4) NMR (1H nuclear magnetic resonance spectroscopy)

Proton NMR spectra were collected in a solution state dissolved in deuterated DMSO using a Bruker 500 MHz NMR spectrometer, and the measurement parameters of each sample are shown in the NMR spectrum (Figure 4).

¹H NMR (500 MHz, DMSO-d₆) δ: 1.47 (d, 6H), 1.80~2.10 (m, 4H), 2.55~2.70 (m, 3H), 2.80 (m, 2H), 3.10 (m, 2H), 3.50 (m, 2H), 4.00 (m, 2H), 5.00 (m, 1H), 5.30 (s, 2H), 7.0 (s, 2H), 7.55-7.66 (m, 2H), 7.80(m, 2H), 10.2 (br, 1H), 12.6 (br, 1H)

### (5) HSM (hot stage microscopy)

Hot stage microscopy was carried out by using a Leica DM LP microscope with a TMS93 controller and a Linkman hot stage (model FTIR 600). Samples were observed by using a lambda plate with a crossed polarizer at either 10×0.22 or 20×0.40 magnification. A sample was placed on a coverslip and another coverslip was placed over the sample. The sample was visually observed as the stage was heated. A drop of mineral oil may be added to the sample in some cases to investigate outgassing. Images were captured by using a SPOT Insight color digital camera with SPOT software v. 4.5.9 (Figure 5).

### (6) DVS (dynamic vapor sorption)

Moisture sorption/desorption data were collected on a DVS Intrinsic instrument (Surface Measurement Systems). Samples were not dried prior to analysis. Sorption and desorption data were collected over the range of 5% to 95% RH with 10% RH increments under nitrogen. The equilibrium criterion used in the analysis was less than 0.0100% change in weight in 5 minutes with a maximum equilibration time of 3 hours. Data on the initial moisture content of the samples were not corrected. The results are represented in Figure 6.

### (7) Stability test (XRPD, naked eye observation, HPLC)

To investigate the stability of Crystalline Form A, after storage for 2 weeks under accelerated (40°C/75%RH) and severe (80°C) conditions, the physical stability was confirmed by naked eye observation and XRD measurement (Figure 7), and chemical stability was confirmed by the analysis of the sample purity using HPLC (Table 3). HPLC equipment and analysis methods are represented in Table 1.

**[Table 1]**

| | | |
|---|---|---|
| HPLC equipment | Agilent 1100 series | |
| Column | YMC-Triart C18 3 µm, 4.6 × 250 mm, YMC | |
| Injection volume | 20 µL | |
| Flow rate | 0.5 mL/min | |
| Column temperature | 10°C | |
| Detection wavelength | 291 nm | |
| Mobile phase A | 25:5:70:0.1 Acetonitrile/Methanol/H₂O/TFA | |
| Mobile phase B | 85:5:10:0.1 Acetonitrile/Methanol/H₂O/TFA | |
| Total running time | 80 minutes | |

| Gradient system | | |
|---|---|---|
| Time (Min) | Mobile phase A (%) | Mobile phase B (%) |
| 0 | 100 | 0 |
| 20 | 35 | 65 |
| 40 | 35 | 65 |
| 42 | 0 | 100 |
| 58 | 0 | 100 |
| 60 | 100 | 0 |
| 80 | 100 | 0 |

### (8) Results

As a result of XRPD analysis, it was confirmed that the compound was in a crystalline form, and specific values are represented in Table 2 below.

**[Table 2]**

| **No.** | **2θ** | **No.** | **2θ** |
|---|---|---|---|
| 1 | 4.4665(8) | 31 | 24.5111(4) |
| 2 | 9.1525(3) | 32 | 24.8838(6) |
| 3 | 9.6679(8) | 33 | 25.3512(9) |
| 4 | 11.7203(7) | 34 | 25.835(1) |
| 5 | 12.1884(2) | 35 | 26.4192(9) |
| 6 | 12.914(2) | 36 | 26.793(2) |
| 7 | 13.5211(4) | 37 | 27.1211(5) |
| 8 | 13.6756(3) | 38 | 27.5957(4) |
| 9 | 14.1779(6) | 39 | 28.096(1) |
| 10 | 14.4812(2) | 40 | 28.636(2) |
| 11 | 15.3705(2) | 41 | 28.901(2) |
| 12 | 15.9270(3) | 42 | 29.599(3) |
| 13 | 17.3194(3) | 43 | 29.932(1) |
| 14 | 17.7033(5) | 44 | 30.319(1) |
| 15 | 17.899(1) | 45 | 30.8748(7) |
| 16 | 18.2125(5) | 46 | 31.2219(7) |
| 17 | 18.399(1) | 47 | 31.544(3) |
| 18 | 18.688(3) | 48 | 32.013(1) |
| 19 | 19.1952(4) | 49 | 32.7866(9) |
| 20 | 19.5251(4) | 50 | 33.0845(9) |
| 21 | 20.6232(5) | 51 | 33.755(2) |
| 22 | 21.058(3) | 52 | 34.222(2) |
| 23 | 21.3409(4) | 53 | 34.511(5) |
| 24 | 21.5596(5) | 54 | 35.280(6) |
| 25 | 22.469(1) | 55 | 36.414(3) |
| 26 | 22.6441(3) | 56 | 37.344(2) |
| 27 | 23.1980(4) | 57 | 38.191(9) |
| 28 | 23.6582(3) | 58 | 38.678(4) |
| 29 | 23.9406(8) | 59 | 38.957(4) |
| 30 | 24.156(2) | 60 | 39.7741 |

According to TGA, a weight loss of about 0.7% was observed from ambient temperature to 100°C. A dramatic change in the slope of the TGA thermogram was observed at about 230°C, which is most likely related to the degradation of the material.

On the result of DSC, an endotherm was observed at about 221°C (onset), which is due to the melting point of the material based on the HSM observation (melting range: 217.1-226.4°C). According to the HSM observation, melting occurred simultaneously with decomposition.

As a result of DVS, there were a weight increase of 2.58% during sorption from 5% to 95%RH and a weight loss of 2.49% during desorption from 95% to 5%RH. The solid retrieved after DVS showed XRPD patterns similar to the initial ones.

Crystalline Form A according to the present invention showed chemical stability for 2 weeks under accelerated condition (40°C, 75% RH) and severe condition (sealed state, 80°C), and physical/chemical stability was confirmed since there was no change in the crystal form in XRPD, as shown in Figure 7.

### Example 3: Preparation of Crystalline Form D

Crystalline Form D was prepared by the following three methods.
(1) Crystalline Form A was dissolved in methanol solvent at 60°C to make a solution, and then filtered with cold methyl tert-butyl ether through a 0.2 µm nylon filter. At this time, the ratio of methanol and methyl tert-butyl ether was 1:4. After filtration with an anti-solvent, a solid was not formed and the solution state was maintained, and the solution was kept in a frozen state for 5 days to obtain a white solid.
(2) Crystalline Form A was dissolved in methanol solvent at 60°C to make a saturated or concentrated solution, filtered through a preheated 0.2 µm nylon filter, and placed in a vial preheated at the same temperature. Then, the obtained product was slowly cooled to room temperature on a heating plate with the heat turned off. In this state, no solid was formed. As such, after cooling to 2-8°C for 3 days, the obtained product was placed in a freezer for 19 days to obtain a solid.
(3) A glass vial containing Crystalline Form A was placed in a vial with a methanol solvent and covered with a lid. Then, the vial was stored at room temperature for 21 days to obtain a white solid.

### Example 4: Analysis of Crystalline Form D

XRPD analysis was carried out in the same manner as in Example 2 (Figure 8).

**[Table 3]**

| **No.** | **2θ** | **No.** | **2θ** |
|---|---|---|---|
| 1 | 4.430(1) | 30 | 25.2570(9) |
| 2 | 8.8729(5) | 31 | 25.5228(6) |
| 3 | 9.378(2) | 32 | 26.0531(6) |
| 4 | 11.714(1) | 33 | 26.747(6) |
| 5 | 11.9179(9) | 34 | 27.0881(9) |
| 6 | 12.5314(2) | 35 | 27.7624 |
| 7 | 13.3955(2) | 36 | 28.032 |
| 8 | 14.0981(4) | 37 | 28.03(2) |
| 9 | 14.2666(8) | 38 | 28.401(6) |
| 10 | 14.574(1) | 39 | 29.366(8) |
| 11 | 15.2248(4) | 40 | 29.576(5) |
| 12 | 15.6420(5) | 41 | 30.109(3) |
| 13 | 16.0851(3) | 42 | 30.587(2) |
| 14 | 16.449(1) | 43 | 30.716(3) |
| 15 | 17.0613(4) | 44 | 31.723(2) |
| 16 | 17.6192(5) | 45 | 32.048(2) |
| 17 | 18.349(4) | 46 | 32.331(2) |
| 18 | 18.9082(4) | 47 | 33.073(1) |
| 19 | 20.6699(4) | 48 | 33.948(2) |
| 20 | 21.0475(4) | 49 | 34.485(7) |
| 21 | 21.451(1) | 50 | 34.863(4) |
| 22 | 22.009(1) | 51 | 35.205(4) |
| 23 | 22.6406(2) | 52 | 36.033(8) |
| 24 | 23.3955(9) | 53 | 36.437(4) |
| 25 | 23.587(1) | 54 | 36.93(3) |
| 26 | 23.995(1) | 55 | 37.335(6) |
| 27 | 24.2236(9) | 56 | 38.349(4) |
| 28 | 24.636(3) | 57 | 38.723(9) |
| 29 | 24.719(2) | 58 | 39.142(8) |

As a result of XRPD analysis, it was confirmed that the compound was in a crystalline form.

### Example 5: Preparation of amorphous form

The amorphous form was prepared by the following three methods.
(1) Crystalline Form A (60 mg) was dissolved in 10 mL of TFE (2,2,2-trifluoroethanol) and filtered through a 0.2 µm PVDF filter into a transparent roundbottom flask. The flask was attached to a rotary evaporator and immersed in a water bath at 60°C, and the solvent was rapidly evaporated to dryness under vacuum. Samples were dried in a vacuum oven at room temperature for 1 day before XRPD test.
(2) Crystalline Form A (810 mg) was dissolved in 120 mL of TFE (2,2,2-trifluoroethanol) and filtered through a 0.2 µm PVDF filter into a transparent roundbottom flask. The flask was attached to a rotary evaporator and immersed in a water bath at 60°C, and the solvent was rapidly evaporated to dryness under vacuum. Samples were dried in a vacuum oven at room temperature for 1 day before XRPD test.
(3) Crystalline Form A (1.03 g) was dissolved in 150 mL of TFE (2,2,2-trifluoroethanol) and filtered through a 0.2 µm PVDF filter into a transparent roundbottom flask. The flask was attached to a rotary evaporator and immersed in a water bath at 60°C, and the solvent was rapidly evaporated to dryness under vacuum. Samples were dried in a vacuum oven at 40°C for 1 day before XRPD test.

### Example 6: Analysis of amorphous form

### (1) XRPD (x-ray powder diffraction)

XRPD analysis was carried out in the same manner as in Example 2 (Figure 9).

### (2) TGA/DSC combination (thermogravimetric analysis/differential scanning calorimetry)

TGA/DSC combination analysis was carried out by using a Mettler-Toledo TGA/DSC 3+ analyzer. The sample was placed in an open aluminum pan, and the pan was sealed. After the pan lid was pierced, the sample was inserted into the TG furnace. Heating under nitrogen was performed from 30°C to a maximum of 250°C at a rate of 10°C/min. The results are represented in Figure 10.

### (3) Temperature Cycling DSC (Differential Scanning Calorimetry)

Temperature cycling DSC was carried out by using a Mettler-Toledo DSC 3+ differential scanning calorimeter. The sample was placed in a sealed aluminum DSC pan, and the weight was accurately recorded. Then, the pan lid was pierced, and the sample was placed into the DSC cell for measurement. The experiment was carried out in the following way under nitrogen. After heating from -30°C to 140°C at a rate of 20°C/min, cooling from 140°C to -30°C at a rate of 20°C/min, and then heating again from -30°C to 200°C at a rate of 20°C/min. The results are represented in Figure 11.

### (4) Stability test (XRPD, naked eye observation, HPLC)

A stability test was carried out in the same manner as in Example 2.

### (5) Results

As a result of XRPD, a halo pattern showing a typical amorphous form without sharp peaks was observed (Figure 9).

As a result of TGA, the sample showed a weight loss of about 4.3% from 49°C to 160°C. A gradual weight loss at 221°C (onset) may indicate a loss of HCl. The weight loss around the stage was about 6.4%, which corresponds to about 1 mole of HCl, assuming the volatile material was HCl.

Temperature cycling DSC was carried out to determine Tg. A broad endotherm at about 69°C (peak) was observed during the initial heating cycle, which is expected due to residual solvent/moisture loss. A step change in the heat flow signal, typically indicative of Tg, was observed at about 89°C during the final heating cycle.

The amorphous form was observed in the form of free-flowing particles that were broken when struck and bubble-like yellow masses with naked eye observation after 2 weeks of severe condition (80°C). Although the amorphous form appeared to be physically stable for at least 2 weeks at 80°C, the amount of impurities in the sample increased under this condition (Table 4).

**[Table 4]**

| Time | Accelerated condition (40°C/75%RH) | Severe condition (80°C) |
|---|---|---|
| Initial | 100.7% | |
| 3 days | 98.8% | 90.7% |
| 1 week | 98.1% | 87.2% |
| 2 weeks | 98.8% | 77.3% |

## Claims

1. A crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof having 3 or more characteristic peaks (2θ) selected from the following X-ray diffraction pattern spectrum:
9.15±0.2°, 12.19±0.2°, 13.68±0.2°, 14.48±0.2°, 15.37±0.2°, 15.93±0.2°, 17.32±0.2°, 19.53±0.2°, 21.56±0.2°, 22.64±0.2°, 23.20±0.2°, 23.66±0.2°, 24.51±0.2°, 27.12±0.2°, 27.60±0.2° and 30.87±0.2°.

2. The crystalline form according to Claim 1, which has 3 or more characteristic peaks (2θ) selected from the following X-ray diffraction pattern spectrum:
9.15±0.1°, 12.19±0.1°, 13.68±0.1°, 14.48±0.1°, 15.37±0.1°, 15.93±0.1°, 17.32±0.1°, 19.53±0.1°, 21.56±0.1°, 22.64±0.1°, 23.20±0.1°, 23.66±0.1°, 24.51±0.1°, 27.12±0.1°, 27.60±0.1° and 30.87±0.1°.

3. A crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof having 3 or more characteristic peaks (2θ) selected from the following X-ray diffraction pattern spectrum:
8.87±0.2°, 12.53±0.2°, 13.40±0.2°, 14.10±0.2°, 15.22±0.2°, 15.64±0.2°, 16.09±0.2°, 17.06±0.2°, 17.62±0.2°, 18.91±0.2°, 20.67±0.2°, 21.05±0.2°, 22.64±0.2°, 23.59±0.2°, 24.22±0.2°, 25.52±0.2°, 26.05±0.2° and 27.09±0.2°.

4. The crystalline form according to Claim 3, which has 3 or more characteristic peaks (2θ) selected from the following X-ray diffraction pattern spectrum:
8.87±0.1°, 12.53±0.1°, 13.40±0.1°, 14.10±0.1°, 15.22±0.1°, 15.64±0.1°, 16.09±0.1°, 17.06±0.1°, 17.62±0.1°, 18.91±0.1°, 20.67±0.1°, 21.05±0.1°, 22.64±0.1°, 23.59±0.1°, 24.22±0.1°, 25.52±0.1°, 26.05±0.1° and 27.09±0.1°.

5. The crystalline form according to Claim 1 or 3, wherein the pharmaceutically acceptable salt is selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid.

6. The crystalline form according to Claim 5, wherein the pharmaceutically acceptable salt is hydrochloric acid.

7. A pharmaceutical composition for the treatment of autoimmune disease including multiple sclerosis, which comprises a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as defined in Claim 1 or 3, together with a pharmaceutically acceptable carrier.

8. A pharmaceutical composition for the prevention or treatment of a disease caused by undesired lymphocyte infiltration related to sphingosine-1-phosphate, which comprises a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as defined in Claim 1 or 3, together with a pharmaceutically acceptable carrier.

9. A pharmaceutical composition for the prevention or treatment of immunoregulation disorder, which comprises a crystalline form of 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as defined in Claim 1 or 3, together with a pharmaceutically acceptable carrier.

10. The pharmaceutical composition according to Claim 9, wherein the immunoregulation disorder is autoimmune disease or chronic inflammatory disease selected from the group consisting of systemic lupus erythematosus, chronic rheumatoid arthritis, inflammatory bowel diseases, multiple sclerosis, amyotrophic lateral sclerosis (ALS), arteriosclerosis, atherosclerosis, scleroderma and autoimmune hepatitis.
